(19) Europäisches Patentamt
European Patent Office
Office européen des brevets

(11) Veröffentlichungsnummer: **0 125 678**
**A1**

(12) # EUROPÄISCHE PATENTANMELDUNG

(21) Anmeldenummer: **84105434.9**

(22) Anmeldetag: **14.05.84**

(51) Int. Cl.³: **C 07 D 307/85**
A 61 K 31/34
//C07C143/78, C07D307/80

(30) Priorität: **17.05.83 DE 3317884**

(43) Veröffentlichungstag der Anmeldung:
**21.11.84 Patentblatt 84/47**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Seuring, Bernhard, Dr.**
**Frankfurter Strasse 19**
**D-6238 Hofheim am Taunus(DE)**

(72) Erfinder: **Lang, Hans-Jochen, Dr.**
**Rüdesheimer Strasse 7**
**D-6238 Hofheim am Taunus(DE)**

(72) Erfinder: **Hropot, Max, Dr.**
**Friedrich-Stolz-Strasse 13**
**D-6093 Flörsheim am Main(DE)**

(72) Erfinder: **Muschaweck, Roman, Dr.**
**Heimchenweg 39**
**D-6230 Frankfurt am Main 80(DE)**

(54) **5-(4-Chlor-3-sulfamoylbenzoyl)-2,3-dihydro-2-benzofurancarbonsäuren und Verfahren zu ihrer Herstellung.**

(57) 5-(4-Chlor-3-sulfamoylbenzoyl)-2,3-dihydro-2-benzofuran-carbonsäuren der Formel I

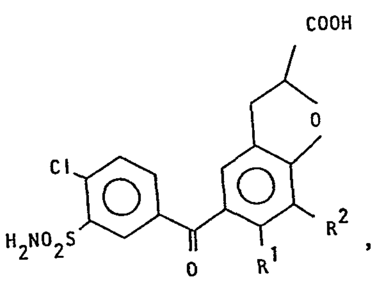

I

in welcher $R^1$, $R^2$ gleich oder verschieden sind und für Wasserstoff, Halogen oder Methyl stehen, sowohl als racemische Gemische wie auch in Form der optischen Isomeren, sowie deren physiologisch verträgliche Salze werden beschrieben; weiterhin ihre Herstellung. Sie sind hervorragende Diuretika und Saluretika mit zusätzlicher urikosurischer Komponente.

EP 0 125 678 A1

Croydon Printing Company Ltd.

5-(4-Chlor-3-sulfamoylbenzoyl)-2,3-dihydro-2-benzofurancarbonsäuren und Verfahren zu ihrer Herstellung

Die Erfindung betrifft 5-(4-Chlor-3-sulfamoylbenzoyl)-2,3-dihydro-2-benzofurancarbonsäure-Derivate der allgemeinen Formel I

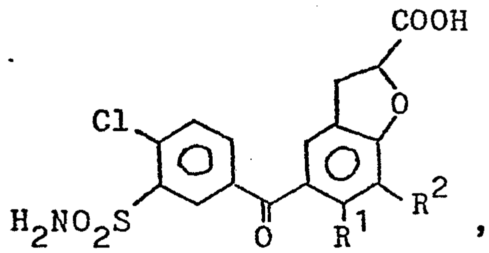 I ,

in welcher
$R^1$, $R^2$ gleich oder verschieden sind und für Wasserstoff, Halogen oder Methyl stehen, sowohl als racemische Gemische wie auch in Form der optischen Isomeren, sowie deren physiologisch verträgliche Salze.

In der Literatur sind bereits mehrere in 4-Stellung acylierte Phenoxyessigsäurederivate bzw. 5-Aroyl-2,3-dihydro-2-benzofurancarbonsäure-Derivate mit salidiuretischer und/oder urikosurischer Wirkung beschrieben (vgl. G.M. Shutske et al., J. Med. Chem. 25, 36 - 44 (1982) und dort zit. Lit.). Alle diese Verbindungen besitzen einen lipophilen Aroylteil und wirken an Ratten kaum oder nur in relativ hohen Dosierungen salidiuretisch. Es war daher sehr überraschend, daß die erfindungsgemäßen Verbindungen der Formel I - die sowohl Phenoxyessigsäure-Derivate als auch Sulfonamide sind - sowohl eine urikosurische als auch eine salidiuretische Wirkung ausüben, und dabei hinsichtlich Aktivität und/oder Wirkdauer die oben erwähnten Verbindungen übertreffen, wie dies durch Versuche an Ratten gelegt werden kann.

Ebenfalls literaturbekannt (deutsche Auslegeschrift 1 129 478) sind Benzophenonsulfonamid-Derivate mit diuretischen und saluretischen Eigenschaften, für die aber keine urikosurischen oder hypourikämischen Effekte nachgewiesen wurden.

Bevorzugte Verbindung ist die 5-(4-Chlor-3-sulfamoylbenzoyl)-6,7-dichlor-2,3-dihydro-2-benzofurancarbonsäure mit $R^1$, $R^2$= Cl.

Die Erfindung betrifft weitherin ein Verfahren zur Herstellung von Verbindungen der Formel I, das dadurch gekennzeichnet ist, daß man

a) ein Carbonsäurederivat der Formel II

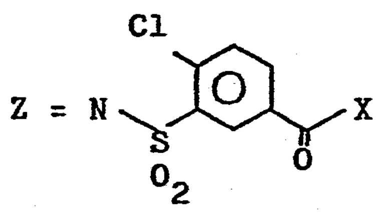 , II

in der Z für zweimal Wasserstoff oder für eine Dimethylaminomethylenschutzgruppe steht und in welcher X für eine Leaving-group, insbesondere aber für Chlor steht, mit einem 2,3-Dihydrobenzofuranderivat der allgemeinen Formel III

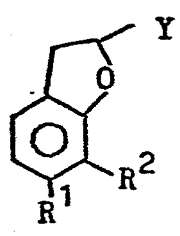 III

in an sich bekannter Weise durch Friedel-Crafts-Acylierung zu Verbindungen der allgemeinen Formel IV umsetzt

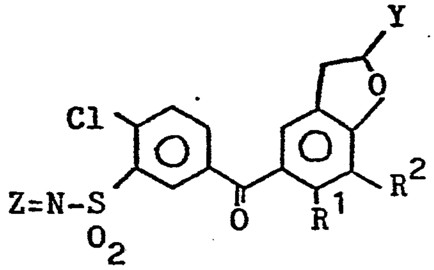 IV ,

in denen $R^1$, $R^2$ und Z die obengenannte Bedeutung besitzen und Y für $\underset{\underset{O}{\|}}{C}-OR^3$ mit $R^3$ in der Bedeutung von H oder

niederes Alkyl mit $C_1$-$C_4$ steht und im Falle, daß Z eine Dimethylaminomethylenschutzgruppe und/oder $R^3$ einen $C_1$-$C_4$-Alkylrest bedeuten, die erfindungsgemäßen Verbindungen der Formel I in literaturbekannter Weise durch Hydrolyse freigesetzt werden,

b) ein 2,3-Dihydrobenzofuranderivat der Formel V

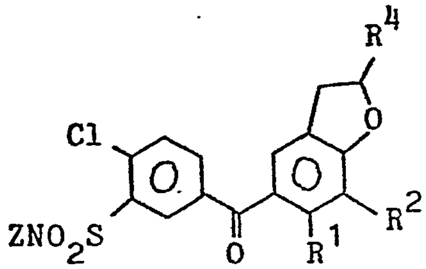

V

,

worin $R^1$, $R^2$ und Z die angegebenen Bedeutungen besitzen und $R^4$ eine $CH_2OH$- oder eine CHO-Gruppe ist, in an sich bekannter Weise mit einem geeigneten Oxidationsmittel in die Carbonsäuren der Formel I überführt.

Verbindungen der Formel IV oder V, die Z in der Bedeutung der Dimethylaminomethylenschutzgruppe enthalten, lassen sich in literaturbekannter Weise (deutsche Offenlegungsschrift 26 54 795; C.A. 89, 108715 (1978)) gegebenenfalls leicht in Verbindungen mit ungeschützter Sulfonamidgruppe (Z gleich zweimal Wasserstoff) überführen und umgekehrt.

Die gemäß Verfahrensweise a) erhaltenen Verbindungen mit Z in der Bedeutung von 2 Wasserstoffatomen und $R^3$ von Wasserstoff sind mit den erfindungsgemäßen Verbindungen der Formel I identisch. Aus den gemäß Verfahrensweise a) erhaltenen Verbindungen der Formel IV, in denen $R^1$ und $R^2$ die angegebene Bedeutung besitzen, $R^3$ für niederes Alkyl und/oder Z für eine Dimethylaminomethylengruppe steht, erhält man die Verbindungen der Formel I durch Hydrolyse im wässerigen Medium in Gegenwart einer Säure oder einer Base, bevorzugt unter Verwendung von Lithium-, Natrium- oder Kaliumhydroxid oder von Salzsäure. Die hydrolytische Spaltung wird auch vorteilhaft durch Zusatz eines polaren organischen Lösungsmittels

wie z. B. von Methanol, Ethanol, Isopropanol, Essigsäure, Tetrahydrofuran durchgeführt. Die Reaktion wird in einem Temperaturbereich zwischen 0 und 120°C durchgeführt, wobei man vorteilhaft zwischen 40 und 100°C arbeitet. Die Herstellung der Verbindungen IV geschieht in an sich bekannter Weise dadurch, daß man die Dihydrobenzofuranderivate der Formel III mit Carbonsäurederivaten der Formel II nach Art, der Friedel-Crafts-Acylierung umsetzt (vgl. Houben-Weyl, Bd. VII/2 a, S. 15 - 62, Georg Thieme Verlag Stuttgart, 1973), wobei als Friedel-Crafts-Katalysator vorzugsweise Aluminiumchlorid Verwendung findet. Alle für Friedel-Crafts-Acylierungen gängigen Lösungsmittel können Verwendung finden, besonders geeignet ist Nitrobenzol, aber auch halogenierte Kohlenwasserstoffe, wie Methylenchlorid oder 1,2-Dichlorethan können vorteilhaft verwendet werden. Die Umsetzungen werden zwischen -20 und 100°C mit 1 - 4 Äquivalenten Katalysator vorgenommen. Zur Vermeidung von Nebenprodukten arbeitet man vorteilhaft bei Temperaturen unterhalb 40°C und stöchiometrischen Mengen Katalysator.

Die verwendeten 2,3-Dihydrobenzofuranderivate der Formel III sind literaturbekannt oder werden analog der in der Literatur angegebenen Verfahrensweise gewonnen (W.F. Hoffmann, O.W. Woltersorf, F.C. Novello und E.J. Cragoe, J.Med. Chem. 24, 865 - 873 (1981)).

Gemäß Verfahrensweise b) wird die Hydroxymethyl- bzw. die Formylgruppe der Verbindungen V in an sich bekannter Weise mit einem geeigneten Oxidationsmittel zur Carboxylfunktion oxidiert und im Falle, daß Z eine Dimethylaminomethylenschutzgruppe bedeutet, diese durch anschließende Hydrolyse, wie unter Verfahrensweise a) näher beschrieben, in die erfindungsgemäßen Verbindungen der Formel I übergeführt. Als bevorzugtes Oxidationsmittel der Hydroxymethylderivate V ($R^4$=$CH_2OH$) wird Pyridiniumdichromat verwendet, wobei zweckmäßigerweise unter Ausschluß von Feuchtigkeit in inerten

polaren organischen Lösungsmitteln, wie Dimethylformamid oder Dimethylacetamid zwischen 0 und 40°C, vorzugsweise bei Zimmertemperatur gearbeitet wird. Prinzipiell können auch andere Oxidationsmittel, wie beispielsweise Kaliumpermanganat in wässeriger NaOH zwischen 10 und 40°C oder Nickelperoxid in wässeriger NaOH, verwendet werden (vgl. Houben-Weyl, Band IV/1b, S. 610 bzw. 845 ff., Georg-Thieme Verlag Stuttgart, 1975).

Die Oxidation kann auch stufenweise so durchgeführt werden, daß die Hydroxymethylverbindungen V mit $R^4$=$CH_2OH$ selektiv zum entsprechenden Aldehyd der Formel V ($R^4$=CHO) oxidiert wird. Als günstiges Oxidationsmittel ist insbesondere aktives Mangandioxidhydrat zu nennen, das vorzugsweise in Methylenchlorid, Acetonitril oder Aceton bei Zimmertemperatur in an sich bekannter Weise zur Anwendung kommt (vgl. Houben-Weyl, Band 7/1, S. 178 - 179 (1954)). Als gleichermaßen günstiges Oxidationsmittel kann Ammoniumhexanitratocerat (IV) in Wasser oder wässeriger Essigsäure bei Temperaturen zwischen 60 und 80°C in literaturbekannter Weise verwendet werden (vgl. Houben-Weyl, Band 4/1b, S. 155, Georg-Thieme-Verlag Stuttgart, 1975)). Die so erhaltenen Aldehyd-Derivate V ($R^4$=CHO) werden sodann in literaturbekannter Weise der weiteren Oxidation zu den entsprechenden Carbonsäuren unterworfen, wobei sich insbesondere Kaliumpermanganat in wässeriger Lösung bei Zimmertemperatur bewährt hat (vgl. C.W. Smith et al., J. Amer. Chem. Soc. 73, 5273 (1951)). In an sich bekannter Weise kann die Oxidation vorteilhaft auch mit Chrom(VI)oxid durchgeführt werden (vgl. Houben-Weyl, Band 4/1b, S. 461, Georg-Thieme-Verlag-Stuttgart, (1974)).

Die als Ausgangskomponente verwendeten 2-Hydroxymethyl-2,3-Dihydrobenzofuranderivate der Formel V können in an sich bekannter Weise (vgl. W.F. Hoffmann, O.W. Woltersdorf, F.C. Novello und E.J. Cragoe, J. Med. Chem. 24, 865 (1981)) dadurch hergestellt werden, daß man Phenole der Formel VI

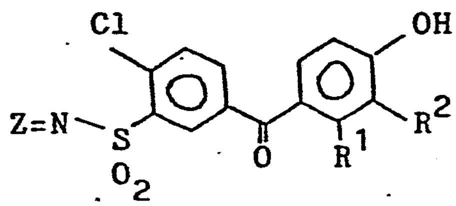

VI

worin $R^1$ und $R^2$ die angegebene Bedeutung haben und Z bevorzugt eine Dimethylaminomethylengruppe ist, mit einem Allylhalogenid in einem organischen Lösungsmittel, wie beispielsweise in Dimethylformamid in Gegenwart einer Base wie z. B. Kaliumcarbonat in die entsprechenden Allylether der allgemeinen Formel VII

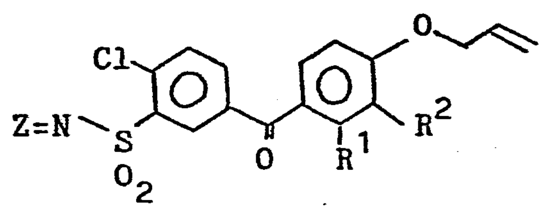

VII

überführt.

Die Alkylether VII können thermisch bei Temperaturen zwischen 180 - 220°C, vorzugsweise in Diphenylether als Lösungsmittel im Sinne einer Claisen-Umlagerung in die 2-Allylphenolderivate der Formel VIII

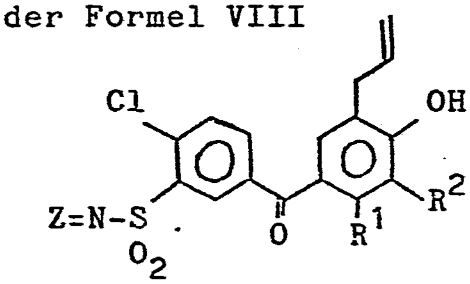

VIII

übergeführt werden, die durch eine Peroxid-Oxidation vorzugsweise unter Verwendung einer organischen Persäure, wie beispielsweise Metachlorperbenzoesäure, in einem organischen inerten Lösungsmittel, wie beispielsweise Methylenchlorid, zwischen 0 und 50°C zu den Verbindungen der Formel V, worin $R^4$ eine $CH_2OH$-Gruppe bedeutet, umgesetzt werden.

Die erfindungsgemäßen Verbindungen der Formel I besitzen in Stellung 2 des Dihydrobenzofuransystems ein Chiralitätszentrum. Durch Behandlung mit einer optisch aktiven Base, wie beispielsweise Brucin oder (-)-Cinchonidin, können in literaturbekannter Weise die Carbonsäuren I in ihre salzartigen Diastereomerenpaare übergeführt und diese durch Kristallisation getrennt werden, aus denen durch Behandlung mit einer Mineralsäure, wie beispielsweise mit wässeriger Salzsäure, die optisch isomeren Carbonsäuren der Formel I freigesetzt werden können.

Erfindungsgemäß können außer der im Experimentalbeispiel beschriebenen 5-(4-Chlor-3-sulfamoylbenzoyl)-6,7-dichlor-2,3-dihydro-2-benzofurancarbonsäure die folgenden erfindungsgemäßen Verbindungen der Formel I dargestellt werden:

5-(4-Chlor-3-sulfamoylbenzoyl)-2,3-dihydro-6,7-dimethyl-2-benzofurancarbonsäure,

5-(4-Chlor-3-sulfamoylbenzoyl)-6-chlor-7-methyl-2,3-dihydro-2-benzofurancarbonsäure,

5-(4-Chlor-3-sulfamoylbenzoyl)-7-chlor-6-methyl-2,3-dihydro-2-benzofurancarbonsäure,

5-(4-Chlor-3-sulfamoylbenzoyl)-6-chlor-2,3-dihydro-2-benzofurancarbonsäure,

5-(4-Chlor-3-sulfamoylbenzoyl)-7-chlor-2,3-dihydro-2-benzofurancarbonsäure.

Die erfindungsgemäßen Verbindungen der Formel I sowie deren physiologisch verträgliche Salze sind Diuretika und Saluretika mit zusätzlicher urikosurischer Komponente. Sie können als Pharmazeutika in der Human- und Veterinärmedizin eingesetzt werden. Dazu werden sie in Dosierungen von 10 - 150 mg/kg Körpergewicht oral, parenteral oder intravenös verabreicht. Für sich allein oder in Kombination mit anderen blutdrucksenkenden, gefäßerweiterenden oder diuretisch wirksamen Substanzen eignen sie sich sowohl zur Behandlung der Hypertonie als auch zur Behandlung von kardial, renal oder hepatisch bedingten Ödemen und anderen auf Störung des Elektrolythaushaltes zurückzuführenden Erscheinungen. Dabei liegt die besondere Bedeutung der obigen Verbindungen in ihrer doppelten Wirksamkeit als Diuretika und Urikosurika. Es ist bekannt, daß während einer diuretischen Behandlung mit bekannten Diuretika in vielen Fällen die Harnsäurekonzentration im Blut eines Patienten steigt. Ein erhöhter Harnsäurespiegel ist ein ernstes Problem bei Gichtpatienten. Darüberhinaus wird ein erhöhter Harnsäurespiegel mehr und mehr als Risikofaktor bei Herzkrankheiten angesehen. Daher kann die diuretische Wirkung mit gleichzeitiger Ausscheidung von Harnsäure als ein Hauptvorteil der erfindungsgemäßen Verbindungen angesehen werden. Die Verbindungen können allein oder in Kombination mit anderen salidiuretisch wirksamen Substanzen auch anderer Wirkungsart angewendet werden. Insbesondere sind zu nennen: Spironolacton, Triamteren, Amilorid und andere $K^+$-retinierende Verbindungen. Aber auch andere rein blutdrucksenkende Verbindungen kommen als mögliche Kombinationspartner in Frage, z. B. Hydralazin, Clonidin, Reserpin und insbesondere Auch beta-blockierende Substanzen wie etwa Metoprolol oder Penbutolol.

Wirksame Mengen der erfindungsgemäßen Verbindungen können einem Patienten auf verschiedene Weise verabreicht werden, z. B. oral in Form von Kapseln oder Tabletten, parenteral in Form von sterilen Lösungen oder Suspensionen und in einigen Fällen auch intravenös als sterile Lösungen.

Die freien Säuren, die selbst wirksam sind, können formuliert werden und aus Gründen der Stabilität, besserer Kristallisierbarkeit, besserer Löslichkeit etc. auch in Form ihrer pharmazeutisch verträglichen Salze verabreicht werden.

Für eine orale Verabreichung können die wirksamen Verbindungen der Erfindung mit einem Verdünnungsmittel oder eßbaren Träger vermischt, in Gelatinekapseln eingeschlossen oder zu Tabletten verpresst werden. Für eine orale therapeutische Verabreichung können die wirksamen Verbindungen in Träger eingearbeitet und in Form von Tabletten, Pastillen, Kapseln, Elixieren, Suspensionen, Syrups, Waffeln, Kaugummi usw. verwendet werden. Diese Präparate sollen mindestens 0,5 Gew.-%, bezogen auf die Gesamtmischung, an wirksamer Substanz enthalten, in Abhängigkeit der besonderen Form kann der Gehalt jedoch zwischen 4 und 70 Gew.-% des Gewichtes der Einheit schwanken. Die Menge an aktiver Verbindung in solchen Präparaten ist so bemessen, daß eine geeignete Dosierung erreicht werden kann. Bevorzugte Mischungen und Präparate enthalten pro oraler Einheitsdosis zwischen 10 und 300 Milligramm der wirksamen Verbindung.

Die Tabletten, Pillen, Kapseln, Pastillen usw. können außerdem die folgenden Bestandteile enthalten:
Bindemittel wie mikrokristalline Cellulose, Tragantgummi oder Gelatine; Träger wie Stärke oder Laktose, Zerfallmittel wie Alginsäure, Maisstärke, usw., Gleitmittel wie Magnesiumstearat oder kolloidales Siliziumdioxid, Süßstoff wie Rohrzucker oder Saccharin, oder einen Aromastoff wie Pfefferminz, Methylsalicylat oder Orangenaroma.
Im Fall einer Einheitsdosis in Kapselform können diese, neben den genannten Substanzen, auch einen flüssigen Träger enthalten, z. B. ein Öl. Tabletten oder Dragees können z. B. auch mit Zucker, Schellack oder anderen darmlöslichen Überzügen versehen sein. Ein Syrup kann neben der wirksamen Verbindung noch Rohrzucker als Süßstoff, bestimmte Konservierungsmittel, Farbstoffe und Geschmackstoffe enthalten.
Die zur Herstellung der Mischungen verwendeten Materialien sollten pharmazeutisch rein und in den zugegebenen Menge ungiftig sein.

Für eine parenteral therapeutische Verabreichung können die wirksamen Verbindungen gemäß der Erfindung in eine Lösung

oder Suspension eingearbeitet werden. Solche Präparate sollen mindestens 0,1 Gew.-% der aktiven Verbindung enthalten, bezogen auf die Gesamtmischung, jedoch kann der Gehalt zwischen 0,5 und 30 Gew.-schwanken. Die Präparate haben einen solchen Gehalt an wirksamer Verbindung, daß eine geeignete Einheitsdosis erhalten werden kann. Die Mischungen und Präparate gemäß der Erfindung enthalten vorzugsweise zwischen 10 und 500 mg aktive Substanz pro parenterale Dosiereinheit.

Die Lösungen und Suspensionen können die folgende Komponente enthalten: ein steriles Verdünnungsmittel, wie Wasser zur Injektion, Salzlösung, nichtflüchtige Öle, Polyethylenglykol, Glycerin, Propylenglykol oder andere synthetische Lösungsmittel, antibakterielle Mittel, wie Benzylalkohol; Antioxydantien wie Ascorbinsäure oder Natriumbisulfit; Chelierungsmittel wie Ethylendiamintetraessigsäure; Puffer, wie Acetate, Zitrate oder Phosphate, und Mittel zur Einstellung der Tonizität wie Kochsalz oder Dextrose. Die parenteralen Präparate können in Ampullen, Einwegspritzen oder Mehrfachdosis-Fläschchen aus Glas oder Plastik abgefüllt werden.

Das folgende Beispiel veranschaulicht die Erfindung.

<u>Beispiel 1</u>

<u>5-(4-Chlor-3-sulfamoylbenzoyl)-6,7-dichlor-2,3-dihydro-2-benzofurancarbonsäure</u>

a) <u>4-Allyloxy-3'-dimethylaminomethylenaminosulfonyl-2,3,4'-trichlorbenzopehenon</u>

Zu einer Mischung aus 13,1 g (30 mMol) 4-Hydroxy-3'-dimethylaminomethylenaminosulfonyl-2,3,4'-trichlorbenzophenon, 5,0 g (36 mMol) gemahlenem Kaliumcarbonat und 50 ml trockenem Dimethylformamid werden 5,6 g (40 mMol) Allylbromid unter magnetischer Rührung bei Zimmertemperatur

zugetropft und ca. 8 Stunden nachgerührt. Die blaßgelbe Suspension wird sodann portionsweise in eine Mischung aus 400 ml Eiswasser und 100 ml 2 N Salzsäure eingerührt und der Niederschlag abfiltriert. Farblose Kristalle, Schmp. 141 - 142°C (aus Aceton/Ethanol).

b) 5-Allyl-4-hydroxy-3'-dimethylaminomethylenaminosulfonyl-2,3,4'-trichlorbenzophenon

16,5 g (35 mMol) 4-Allyloxy-3'-dimethylaminomethylenaminosulfonyl-2,3,4'-trichlorbenzophenon werden in 200 ml Diphenylether über 1 Stunde auf 210 bis 215°C erhitzt und die noch warme Mischung portionsweise unter Rührung in 1,2 l Petrolether eingegossen. Man filtriert den Niederschlag ab, wäscht ihn mehrmals mit Petrolether und kristallisiert aus Methanol um. Farblose Kristalle, Schmp. 190 - 193°C.

c) 6,7-Dichlor-5-(4-chlor-3-dimethylaminomethylenaminosulfonylbenzoyl)-2,3-dihydro-2-hydroxymethylbenzofuran

14,7 g (31 mMol) 5-Allyl-4-hydroxy-3'-dimethylaminomethylenaminosulfonyl-2,3,4'-trichlorbenzophenon werden unter Feuchtigkeitsausschluß in 60 ml Methylenchlorid suspendiert und unter Kühlung im Eisbad tropfenweise mit einer Lösung von 7,4 g (43 mMol) 90 %iger Metachlorperbenzoesäure in 60 ml Methylenchlorid versetzt. Man rührt noch etwa eine Stunde im Eisbad nach und erwärmt sodann kurz auf 35 bis 40°C, wobei eine klare Lösung erhalten wird. Nach Stehenlassen über Nacht bei Zimmertemperatur versetzt man mit weiteren 0,7 g Metachlorperbenzoesäure, und erwärmt nach 4-stündigem Rühren bei Zimmertemperatur nochmals auf 35°C. Man filtriert den Niederschlag ab, wäscht diesen zweimal mit je 10 ml Methylenchlorid und sodann mehrfach mit wässeriger Natriumhydrogencarbonat-Lösung bis zur alkalischen Reaktion. Die organische Phase wird über Natriumsulfat

getrocknet, das Lösungsmittel abdestilliert und das amorphe Produkt durch Chromatographie an Kieselgel mit Toluol-Essigester (3 : 1 bis 1 : 3) gereinigt. Nach Abdestillieren des Lösungsmittels und Umkristallisation aus wenig Methanol erhält man farblose Kristalle, Schmp. 180 - 182°C.

d) 5-(4-Chlor-3-sulfamoylbenzoyl)-6,7-dichlor-2,3-dihydro-2-benzofurancarbonsäure

1,97 g (4 mMol) 6,7-Dichlor-5-(4-chlor-3-dimethylaminomethylenaminosulfonylbenzoyl)-2,3-dihydro-2-hydroxymethylbenzofuran werden unter Feuchtigkeitsausschluß und Inertgasatmosphäre in 10 ml wasserfreiem Dimethylformamid gelöst und mit 5,3 g (14 mMol) Pyridiniumdichromat versetzt. Die dunkelrote Lösung wird einen Tag bei Zimmertemperatur gerührt. Nach Zugabe eiens weiteren Gramms Pyridiniumdichromat läßt man nochmals 24 Stunden bei Zimmertemperatur rühren, gießt sodann in 0,5 N HCl, extrahiert mehrmals mit Essigester und wäscht die vereinigten organischen Phasen mehrfach mit verdünnter Salzsäure und Wasser. Nach Trocknung über Natriumsulfat und Abdestillieren des Lösungsmittels chromatographiert man das stark chromathaltige Produkt an Kieselgel mit einem 10 : 6 : 1 : 2 Gemisch aus Methylenchlorid/Cyclohexan/Eisessig/Ethanol. Die nach Verdampfen des Lösungsmittels erhaltene 5-(4-Chlor-3-dimethylaminomethylenaminosulfonylbenzoyl)-6,7-dichlor-2,3-dihydro-2-benzofurancarbonsäure wird ohne weitere Reinigung in einer Mischung aus 2 ml Eisessig, 0,5 ml konz. Salzsäure und 1 ml Wasser 6 Stunden auf dem Dampfbad erhitzt und sodann im Eisbad gekühlt. Man filtriert den Niederschlag ab und kristallisiert aus Eisessig/Wasser unter Verwendung von Aktivkohle um. Farblose Kristalle, Schmp. 218 - 222°C.

Patentansprüche:

1. 5-(4-Chlor-3-sulfamoylbenzoyl)-2,3-dihydro-2-benzofurancarbonsäuren der allgemeinen Formel I

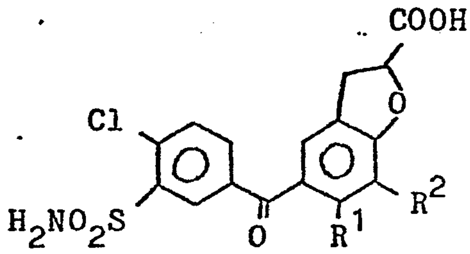

I

in Form ihrer racemischen Gemische wie auch deren optische Isomere, worin $R^1$, $R^2$ gleich oder verschieden sind und für Wasserstoff, Halogen oder Methyl stehen, sowie deren physiologisch verträgliche Salze.

2. 5-(4-Chlor-3-sulfamoylbenzoyl)-6,7-dichlor-2,3-dihydro-2-benzofurancarbonsäure.

3. Verfahren zur Herstellung von Verbindungen gemäß Anspruch 1, dadurch gekennzeichnet, daß man

a) ein Carbonsäurederivat der Formel II

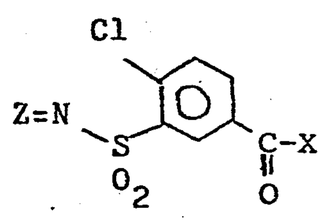

II ,

in der Z für zweimal Wasserstoff oder für eine Dimethylaminomethylenschutzgruppe steht, und in welcher X eine Leaving-group, insbesondere aber Chlor bedeutet, mit einem 2,3-Dihydrobenzofuranderivat der allgemeinen Formel III

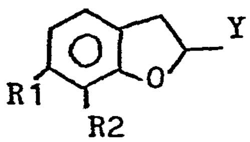

III

in an sich bekannter Weise durch Friedel-Crafts-Acylierung zu Verbindungen der allgemeinen Formel IV umsetzt

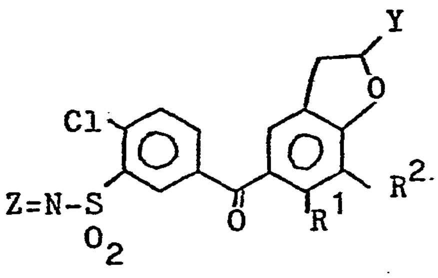

IV

worin $R^1$, $R^2$ und Z die obengenannte Bedeutung besitzen und Y für $-COOR^3$ mit $R^3$ in der Bedeutung von Wasserstoff oder niederes Alkyl mit 1 - 4 C-Atomen steht und im Falle, daß Z eine Dimethylaminomethylen-schutzgruppe und/oder $R^3$ einen Alkylrest bedeuten, die erfindungsgemäßen Verbindungen der Formel I durch Hydrolyse in an sich bekannter Weise erhalten werden,

b) ein 2,3-Dihydrobenzofuranderivat der Formel V

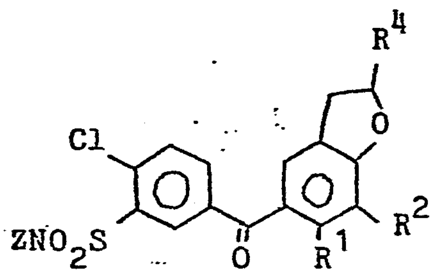

V

worin $R^1$, $R^2$ und Z die angegebenen Bedeutungen besitzen und $R^4$ eine $CH_2OH$- oder eine CHO-Gruppe ist, in an sich bekannter Weise mit einem geeigneten Oxidationsmittel behandelt und im Falle, daß Z eine Dimethylaminomethylenschutzgruppe bedeutet, diese in an sich bekannter Weise hydrolytisch abspaltet,

und gegebenenfalls die nach Weg a) und b) erhaltenen Verbindungen der allgemeinen Formel I mit organischen oder anorganischen Basen in ihre Carbonsäuresalze oder Salze mit Mineralsäuren in die freien Carbonsäuren der Formel I überführt.

4. Pharmazeutische Präparate mit diuretischer, saluretischer und urikosurischer Wirkung bestehend aus bzw. enthaltend eine Verbindung gemäß Anspruch 1.

5. Verfahren zur Herstellung pharmazeutischer Präparate mit diuretischer, saluretischer und urikosurischer Wirkung, dadurch gekennzeichnet, daß man eine Verbindung gemäß Anspruch 1 gegebenenfalls mit pharmazeutischen Trägern und/oder Stabilisatoren in eine für therapeutische Zwecke geeignete Anwendungsform bringt.

HOE 83/F 084

0125678

Patentansprüche für Österreich:

1. Verfahren zur Herstellung von Verbindungen der Formel I

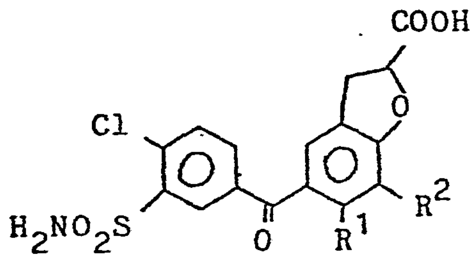

I

in Form ihrer racemischen Gemische wie auch deren optische Isomere, worin R1, R2 gleich oder verschieden sind und für Wasserstoff, Halogen oder Methyl stehen, sowie deren physiologisch verträglichen Salze, dadurch gekennzeichnet, daß man

a) ein Carbonsäurederivat der Formel II

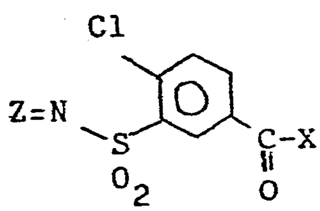

II

in der Z für zweimal Wasserstoff oder für eine Dimethylaminomethylenschutzgruppe steht, und in welcher X eine Leaving-group, insbesondere aber Chlor bedeutet, mit einem 2,3-Dihydrobenzofuranderivat der allgemeinen Formel III

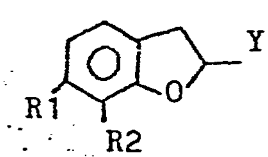

III

in an sich bekannter Weise durch Friedel-Crafts-Acylierung zu Verbindungen der allgemeinen Formel IV umsetzt

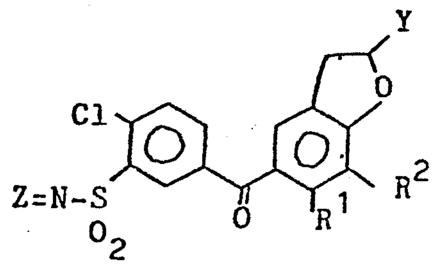

IV

worin R1, R2 und Z die obengenannte Bedeutung besitzen und Y für -COOR3 mit R3 in der Bedeutung von Wasserstoff oder niederes Alkyl mit 1-4 C-Atomen steht und im Falle, daß Z eine Dimethylaminomethylenschutzgruppe und/oder R3 einen Alkylrest bedeuten, die erfindungsgemäßen Verbindungen der Formel I durch Hydrolyse in an sich bekannter Weise erhalten werden,

b) ein 2,3-Dihydrobenzofuranderivat der Formel V

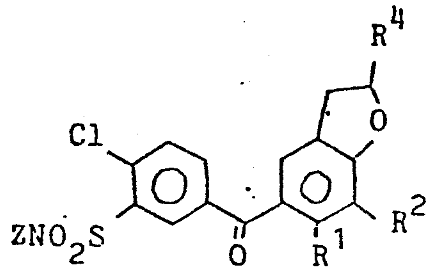

V

worin R1, R2 und Z die angegebenen Bedeutungen besitzen und $R^4$ eine $CH_2OH$- oder eine CHO-Gruppe ist, in an sich bekannter Weise mit einem geeigneten Oxidationsmittel behandelt und im Falle, daß Z eine Dimethylaminomethylenschutzgruppe bedeutet, diese in an sich bekannter Weise hydrolytisch abspaltet,

und gegebenenfalls die nach Weg a) und b) erhaltenen Verbindungen der allgemeinen Formel I mit organischen oder anorganischen Basen in ihre Carbonsäuresalze oder

Salze mit Mineralsäuren in die freien Carbonsäuren der Formel I überführt.

2. Verfahren nach Anspruch 1, dadurch gekennzeichnet, daß $R^1$ und $R^2$ Chlor bedeuten.

3. Verfahren zur Herstellung pharmazeutischer Präparate mit diuretischer, saluretischer und urikosurischer Wirkung, dadurch gekennzeichnet, daß man eine nach Anspruch 1 erhaltene Verbindung gegebenenfalls mit pharmazeutischen Trägern und/oder Stabilisatoren in eine für therapeutische Zwecke geeignete Anwendungsform bringt.

Europäisches Patentamt

# EUROPÄISCHER RECHERCHENBERICHT

0125678
Nummer der Anmeldung

EP 84 10 5434

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,Y | DE-B-1 129 478 (J.R. GEIGY) * Insgesamt * | 1-5 | C 07 D 307/85<br>A 61 K 31/34 //<br>C 07 C 143/78<br>C 07 D 307/80 |
| Y | DE-A-2 630 800 (MERCK) * Insgesamt * | 1-5 | |
| P,Y | EP-A-0 104 483 (HOECHST AG) * Insgesamt * | 1-5 | |

RECHERCHIERTE SACHGEBIETE (Int. Cl. 3)

C 07 D 307/00

Der vorliegende Recherchenbericht wurde für alle Patentansprüche erstellt.

| Recherchenort | Abschlußdatum der Recherche | Prüfer |
|---|---|---|
| DEN HAAG | 09-08-1984 | ALLARD M.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82